# EUROPEAN PATENT SPECIFICATION

(11) **EP 4 447 856 B1**
(45) Date of publication and mention of the grant of the patent: **06.05.2026**
(21) Application number: 22839453.2
(22) Date of filing: 05.12.2022
(51) Int. Cl.: A61D 7/00, A61M 5/315

(54) **MULTI-DOSE SYRINGE AND METHOD OF MULTIPLE DOSING A LIQUID OR SEMILIQUID PRODUCT**
MEHRFACHDOSIERUNGSSPRITZE UND VERFAHREN ZUR MEHRFACHDOSIERUNG EINES FLÜSSIGEN ODER HALBFLÜSSIGEN PRODUKTS
SERINGUE MULTI-DOSE ET PROCÉDÉ DE DOSAGE MULTIPLE D'UN PRODUIT LIQUIDE OU SEMI-LIQUIDE

(30) Priority: 16.12.2021 IT 202100031520
(43) Date of publication of application: 23.10.2024
(73) Proprietor: Platinum Pharma Service S.r.l., 65013 Città Sant'Angelo (PE) (IT)
(72) Inventor: RICCI, Alfredo, 65013 Città Sant'Angelo - PE (IT)
(74) Representative: Di Bernardo, Antonio
(86) International application number: PCT/IB2022/061774
(87) International publication number: WO 2023/111766

(56) References cited:
- US-A1- 2008 071 227
- US-A1- 2021 146 058

## Description

### TECHNICAL FIELD

The present invention generally relates to the technical field of dosing syringes. More particularly, the present invention relates to a syringe, preferably for veterinary use, configured to administer precise and over time repeatable doses of a liquid or semiliquid product, for example a medicine or dietary supplement, contained therein, and a method of multiple dosing a product. The multi-dose syringe according to the present invention finds further application in the food sector, or in any other sector where it is necessary to administer multiple and repeatable doses of the same liquid or semiliquid product.

### BACKGROUND

Dosing syringes, of the pre-filled or fillable type, are widely used in the veterinary field for feeding animals, especially pets, but not only.

They represent a growing market, as they effectively reduce errors in dosing a product to be administered. In fact, once filled, or purchased pre-filled with the desired product, such as a medicine or dietary supplement, they can easily be used to dose, and subsequently administer, the correct dose for a specific animal.

As is well known, in the veterinary field, the dosage of a medicine or dietary supplement to be administered to an animal varies considerably depending on the animal's weight.

Disposable syringes are usually used to administer a dose of the product to an individual animal. In this case, the veterinarian selects the appropriate dose on the syringe for the particular animal to be treated, dispenses the selected dose and disposes of the syringe with the remaining product content inside. Alternatively, when a product is to be administered to a large number of animals, multiple doses can be contained in a single syringe and the veterinarian selects each time the appropriate dose for the particular animal to be treated on the syringe and dispenses the selected dose.

It follows that dosing syringes must be capable of administering doses of varying amounts, consistent with the weight of the animal to be treated.

A dispensing syringe according to the prior art is described, for example, in the international patent application no. WO 02/26298 A2. This dosing syringe comprises a cylindrical body configured to contain a product to be administered in doses. The cylindrical body has an opening at one end for dispensing a dose of product, while the other end is adapted to receive a plunger that is longer than the length of the cylindrical body and slides inside the cylindrical body. The plunger has a series of indentations along its entire length. The dispensing syringe also includes a movable dose selection ring, which is arranged around the plunger and can be rotated about the longitudinal axis of the plunger. In particular, the ring is screwed around the plunger, as the indentations on the plunger form a kind of thread. The movable dose selection ring comprises calibration means, formed on a sleeve and adapted to set the desired dose by rotating the movable ring until the desired dose is reached, and means of engaging the movable ring with the plunger, such that the movable ring moves integrally with the plunger until it abuts against the cylindrical body of the syringe, so as to dispense the desired set dose. Document US 2021/146058 A1 also discloses a dispensing syringe.

However, known dispensing syringes of the type described above have the following drawbacks.

Firstly, whenever the user wishes to administer a dose, he must set it by acting on the movable ring, i.e. turning it along a thread, as briefly described above. This is particularly disadvantageous when the dose to be administered is always the same, e.g. in the case of administering equal doses of a medicine to the same animal at different times of the day or in the case of administering the same dose to different animals with the same or similar body weight. In this case, in addition to performing an unnecessary operation, the operator also runs the risk of making a mistake in setting the dose. Moreover, the longitudinal positioning of the movable ring by means of a thread is a time-consuming operation.

Secondly, when setting a dose, the animal may move, causing an unwanted rotation of the movable dose selection ring during the administration of the product. This is disadvantageous as it would compromise the accuracy of the previously set dose. In fact, each rotation of the movable ring corresponds to a different dose to be administered.

Other dosing syringes known in the state of the art involve an insert, which inserts/disconnects into/from the plunger, with similar problems to those described above.

### OBJECTS AND SUMMARY OF THE INVENTION

The main object of the present invention is therefore to provide a multi-dose syringe and method of multiple dosing a liquid or semiliquid product that can overcome the drawbacks mentioned above with reference to dosing syringes of the known type.

More specifically, the main object of the present invention is to provide a multi-dose syringe and a method of multiple dosing a liquid or semiliquid product, such that precise and repeatable doses of the product contained in the syringe or even doses with different volumes can be administered.

Another object of the present invention is to provide a multi-dose syringe and a method of multiple dosing a liquid or semiliquid product, such as to enable the dose to be administered can be varied quickly and immediately.

Yet another object of the present invention is to provide a multi-dose syringe and method of multiple dosing a liquid or semiliquid product, such that dosing errors are avoided, either by the user or due to external causes unrelated to the user, e.g. impact with an animal or other stresses.

Yet another object of the present invention is to provide a multi-dose syringe, configured in such a way as to avoid loss of time in the dose-setting phase, whether they are doses with constant volumes or doses with variable volumes.

Yet another object of the present invention is to provide a multi-dose syringe that is structurally simple and easily assembleable.

Last but not least, another object of the present invention is to provide a multi-dose syringe, which can be produced with competitive times and costs.

These and other objects of the present invention are achieved by a multi-dose syringe and a method of multiple dosing a liquid or semiliquid product incorporating the features of the accompanying claims, which form an integral part of the present description.

The invention thus relates, in a first aspect thereof, to a multi-dose syringe comprising a hollow cylindrical body configured to contain a product to be administered in a dosed manner and from which a spout extends, which is provided with a product outlet opening, and a plunger, sliding inside the hollow cylindrical body.

A helical indentation is formed on an outer surface of the plunger, which comprises a plurality of teeth, each having a first and a second longitudinal surface and a first and a second transverse surface, extending, parallel to each other, orthogonally from a respective longitudinal surface and the syringe further comprises a flanged dose-setting sleeve, irremovably mounted on one end of the hollow cylindrical body for the entry of the plunger, and a dose-setting ring mounted on the flanged dose-setting sleeve and rotatable with respect thereto and with respect to the plunger.

A series of seats and at least one tooth configured to be housed with interference into a respective seat are provided on the facing surfaces of the flanged sleeve and ring, respectively.

The plunger can be rotated with respect to the hollow cylindrical body until the first longitudinal surface of a tooth abuts against a ring abutment surface projecting towards the inside of the hollow cylindrical body, thereby setting a predetermined dose of the product to be administered.

Due to such a combination of features, in particular due to the aforementioned interference coupling between the flanged dose-setting sleeve and the dose-setting ring and the interaction of the helical indentation of the plunger with the dose-setting ring, the syringe according to the invention allows for predetermined and repeated doses of a product to be administered in a simple and immediate way. Furthermore, once the dose to be administered has been set, it cannot be changed accidentally, e.g. due to the movement of an animal, but only the user can vary the dose, if desired.

In one embodiment, the flanged dose-setting sleeve comprises a first annular body for mounting the flanged sleeve on the end of the hollow cylindrical body for the entry of the plunger, a second annular body provided with an opening and a flange interposed between the first annular body and the second annular body, the housing seats with interference of at least one tooth of the dose-setting ring being formed on a surface of the flange.

In one embodiment, there is a stop element on an inner surface of the second annular body, against which the first transverse surface of a tooth of the helical indentation abuts, thereby interrupting the stroke of the plunger inside the hollow cylindrical body, when administering the dosed amount of product.

In one embodiment, the plunger comprises a rubber associated with one end of the plunger intended to slide inside the hollow cylindrical body, a connector being interposed between the rubber and the end of the plunger to allow for rotation of the plunger with respect to the rubber inside the hollow cylindrical body.

In one embodiment, the dose-setting ring comprises a main body, from which a tab extends outwards, the interference housing seats of the at least one tooth of the dose-setting ring being formed at a surface of the tab.

In one embodiment, the tab extends outwards from a portion of the dose-setting ring, which has a reduced thickness, preferably comparable to the thickness of the tab. The tab is therefore, advantageously, more flexible and therefore easier for the user to handle when setting a dose of product to be administered.

In one embodiment, the dose-setting ring includes at least one, preferably two recesses, of localised weakening of the dose-setting ring.

In one embodiment, the abutment surface of a tooth of the plunger indentation is formed on an extension of the tab facing towards the inside of the dose-setting ring.

In one embodiment, a stop extends from a flange surface facing the second annular body to limit the rotation of the dose-setting ring with respect to the flanged dose-setting sleeve or to exclude the dose-setting ring from cooperation with the flanged dose-setting sleeve.

In one embodiment, progressive numbers, one for each seat, are marked at the flanged sleeve seats, each indicating a quantity, preferably expressed in millilitres, of product to be dosed and subsequently administered in a dosed manner.

In one embodiment, there is a handle at one end of the plunger, preferably with a faceted outer surface.

In one embodiment, the spout is preferably made in one piece with the hollow cylindrical body, or is a separate piece subsequently assembled on the hollow cylindrical body and is preferably closed with a plug. Making the spout as a separate piece has the advantage of reducing the dimensions of the multi-dose syringe during transport and consequently shipping and packaging costs. Furthermore, a multi-dose syringe without a spout can be advantageously pre-filled using any known filling machine.

In a second aspect thereof, the invention relates to a non-therapeutic method of multiple dosing a product conducted by using the multi-dose syringe defined above, the method comprising the following steps of:
- rotating a dose-setting ring with respect to a flanged dose-setting sleeve irremovably mounted on one end of a hollow cylindrical body of the multi-dose syringe until at least one tooth of the ring is positioned at a respective seat of the flanged sleeve, corresponding to a predetermined volume of product to be dosed;
- interference fitting the tooth and the seat;
- rotating a plunger with respect to the flanged sleeve - dose-setting ring assembly, until a first longitudinal surface of a tooth of a helical indentation formed on an outer surface of the plunger abuts against a ring abutment surface, projecting towards the inside of the dose-setting ring, thereby setting a predetermined dose of the product to be administered.

In one embodiment, the method further comprises, downstream of the plunger rotation step, a step of pressing the plunger towards the inside of the hollow cylindrical body, until a second transverse surface of a tooth of the helical indentation abuts against a stop element formed in the flanged sleeve, so that the plunger's stroke inside the hollow cylindrical body is interrupted when the dosed amount of product is administered.

In one embodiment, the method comprises the following further steps of setting a new dose of product to be administered:
- lifting the ring from the flanged sleeve, so that the tooth is released from the seat;
- rotating the ring with respect to the flanged sleeve until at least one tooth of the ring is positioned at a respective seat of the flanged sleeve corresponding to a new dose to be administered;
- interference fitting the tooth and the seat; and
- rotating a plunger with respect to the flanged sleeve - ring assembly, until a first longitudinal surface of a tooth of a helical indentation of the plunger abuts against the ring abutment surface, projecting towards the inside of the dose-setting ring, thereby setting a predetermined dose of the product to be administered.

Further features and advantages of the present invention will be more evident from the description of the accompanying drawings.

### BRIEF DESCRIPTION OF THE DRAWINGS

The invention is described hereinbelow with reference to some examples provided by way of non-limiting example and illustrated in the accompanying drawings. These drawings illustrate different aspects and embodiments of the present invention and reference numerals illustrating structures, components, materials and/or similar elements in different drawings are indicated by similar reference numerals, where appropriate.
- Figure 1 is a perspective view of a multi-dose syringe according to a first preferred embodiment of the present invention;
- Figure 2 is an exploded perspective view of the multi-dose syringe of Figure 1;
- Figure 3 is a longitudinal section view of the multi-dose syringe of Figure 1;
- Figure 4 is a partial perspective view of a plunger of the multi-dose syringe of Figure 1;
- Figure 5 is a perspective view, taken from another angle, of the plunger of Figure 4;
- Figure 5A is an enlarged view of the detail circled in Figure 5;
- Figure 6 is a perspective view of a flanged dose-setting sleeve of the multi-dose syringe of Figure 1;
- Figure 7 is a perspective view from above of a dose-setting ring of the multi-dose syringe of Figure 1;
- Figure 8 is a perspective view from below of the dose-setting ring of Figure 7;
- Figure 9 is a perspective view from above of an alternative embodiment of a dose-setting ring of the multi-dose syringe of Figure 1;
- Figure 10 is a perspective view from below of the dose-setting ring of Figure 9;
- Figure 11 is a partial perspective view of the multi-dose syringe of Figure 1, in an operational step in which the dose to be administered has been set and the plunger is about to be rotated to prepare the syringe for dispensing the set dose;
- Figure 12 is a partial perspective view of the multi-dose syringe of Figure 1, showing an operational step in which the dose to be administered has been set, the plunger has been rotated and is ready to be pressed to allow the previously set product dose to be administered;
- Figure 13 is a partial perspective view of the multi-dose syringe of Figure 1, showing the multi-dose syringe of Figure 1 in the step of completing the administration of a pre-set dose; and
- Figure 14 is a partial perspective view of the multi-dose syringe of Figure 1, showing an operational step in which the dose-setting ring is excluded from cooperation with the flanged dose-setting sleeve and the plunger can rotate freely to administer a measured, and not pre-set, dose of liquid or semiliquid product.

### DETAILED DESCRIPTION OF THE INVENTION

While the invention is susceptible to various modifications and alternative constructions, certain preferred embodiments are shown in the drawings and are described hereinbelow in detail. It must in any case be understood that there is no intention to limit the invention to the specific embodiment illustrated, but, on the contrary, the invention intends covering all the modifications, alternative and equivalent constructions that fall within the scope of the invention as defined in the claims.

The use of "for example", "etc.", "or" indicates non-exclusive alternatives without limitation, unless otherwise indicated. The use of "includes" means "includes, but not limited to" unless otherwise indicated.

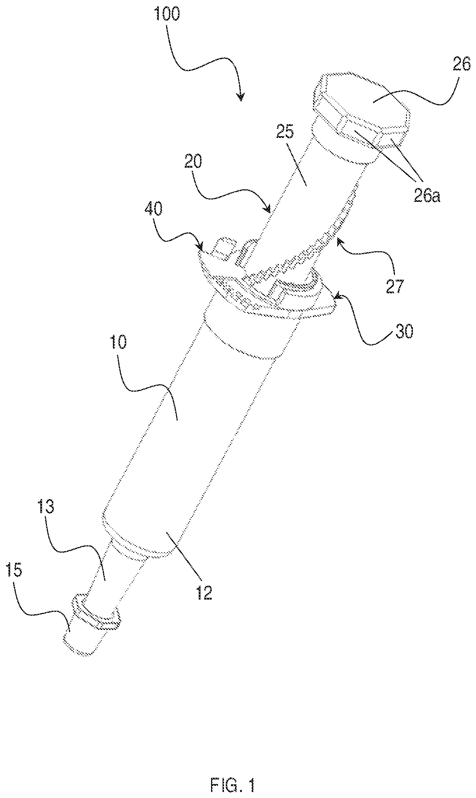

With reference to Figures 1 to 3, they show a multi-dose syringe according to a preferred embodiment of the present invention.

The multi-dose syringe, generally referred to by the reference number 100, is preferably a pre-filled syringe, but it may also be filled at the time of administration, and comprises a hollow cylindrical body or barrel 10, either transparent or opaque, containing a liquid or semiliquid product, e.g. a medicine or dietary supplement, to be dosed and administered to an animal, for example.

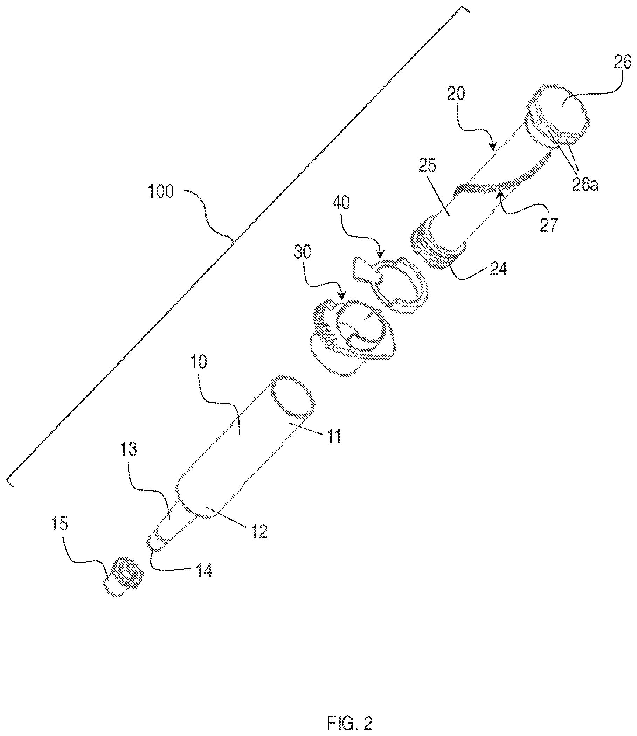

As can be seen in Figure 2, the hollow cylindrical body 10 has an end 11 for insertion of a plunger 20 and another end 12, opposite the end 11 for insertion of the plunger 20, from which a spout 13 extends, hollow and substantially frustoconical, provided with an opening 14 for the outlet of the product being administered.

The spout 13 is preferably made in one piece with the hollow cylindrical body 10 and is preferably closed with a plug 15. In an alternative embodiment, the spout 13 is a separate element, which is assembled on the hollow cylindrical body 10 when the dosing syringe 100 is used. This advantageously saves on packaging material and reduces space during storage and transport of the multi-dose syringe.

As will be described in detail below, the plunger 20 is configured to rotate about a longitudinal axis of the multi-dose syringe 100 and to slide inside the hollow cylindrical body 10, respectively, to set a dose of the product contained in the syringe and subsequently administer the set dose in a precise and repeatable manner over time.

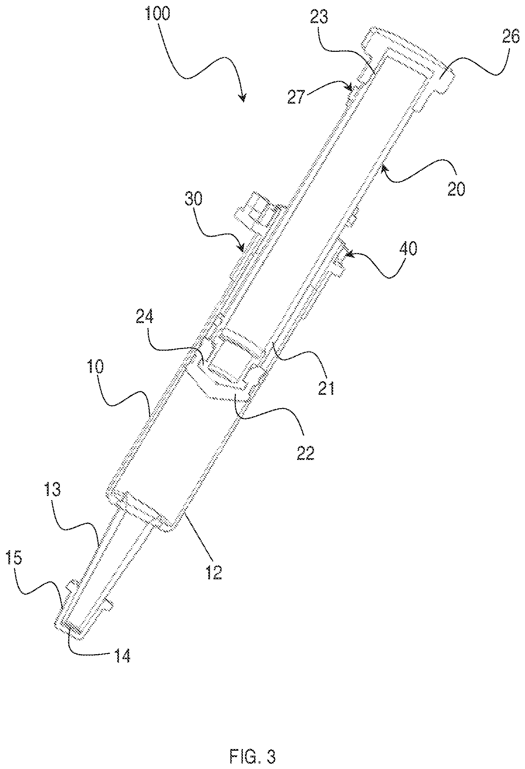

As can be seen in detail in Figure 3, the plunger 20 consists of a hollow cylinder, which carries a rubber 22 at one end 21 thereof intended to slide inside the hollow cylindrical body 10 of the syringe 100. When the plunger 20 is pressed by the user to administer a dosed amount of product, the rubber 22 compresses the product, while at the same time creating a seal with an inner surface of the hollow cylindrical body 10. In other words, the rubber 22 ensures that, during administration, the product contained in the hollow cylindrical body 10 of the multi-dose syringe 100 is directed towards the product outlet spout 13 and not in the opposite direction.

Preferably, a connector 24 is interposed between the end 21 of the plunger 20 and the rubber 22, the function of which, as will be described in greater detail below, is to allow a rotation of the plunger 20 with respect to the rubber 22, while ensuring the fluid seal as the plunger 20 slides inside the hollow cylindrical body 10 of the multi-dose syringe 100. The connector 24 can be a separate component, mounted on the end 21 of the plunger 20, or it can be made as a single piece with the plunger 20.

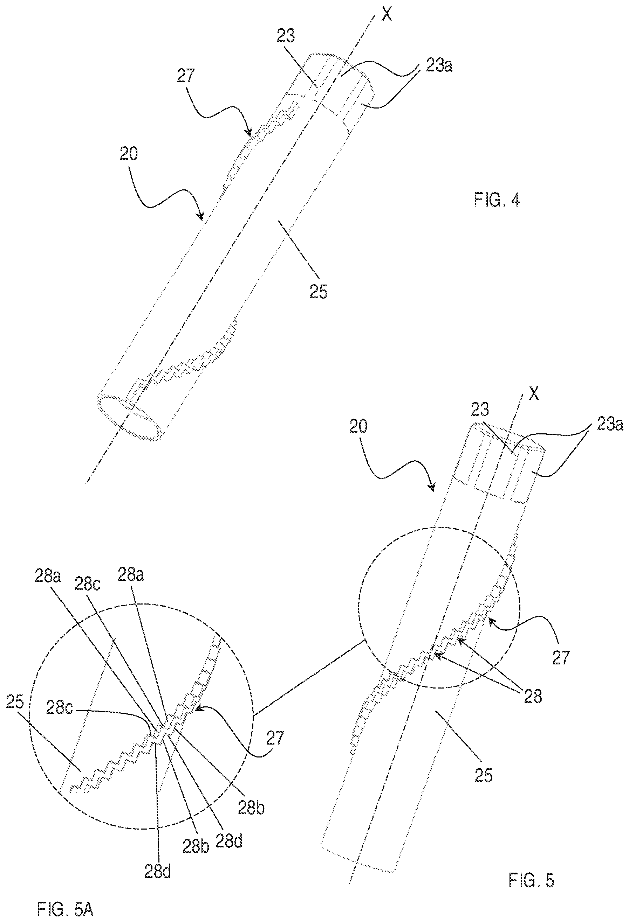

At another end 23 of the plunger 20, opposite the end 21 carrying the rubber 22, there is a handle 26, on which the user acts with his thumb to push the plunger 20 into the hollow cylindrical body 10 when administering a pre-set dose of product. In the illustrated embodiment, the handle 26 is a separate element, which is mounted on the end 23 of the plunger 20, permanently and without any relative movement between the handle 26 and the plunger 20. For this purpose, and as shown in detail in Figures 4 and 5, the end 23 is faceted, with a number of flat faces 23a corresponding to the number of flat faces (not shown) formed at an inner surface of the handle 26.

Preferably, the handle 26 has a faceted outer surface comprising a number of flat faces 26a (Figures 1 and 2), e.g. eight flat faces 26a. This faceted outer surface advantageously allows for a better grip of the handle 26 by the user.

With reference to Figures 4, 5 and 5A, on an outer surface 25 of the plunger 20 a helical indentation 27 is formed, comprising a plurality of teeth 28, which extends over the entire outer surface 25 or, alternatively, over part of it. The teeth 28 have a similar pattern to that of a spiral staircase.

As shown in detail in Figure 5A, each tooth 28 of the helical indentation 27 has a first longitudinal surface 28a and a second longitudinal surface 28b, which extend, parallel to each other, along a longitudinal axis X (shown for example in Figure 5) of the plunger 20, and a first transverse surface 28c and a second transverse surface 28d, which extend, parallel to each other, orthogonally from a respective longitudinal surface 28a, 28b, to form a respective tooth 28 of the helical indentation 27. The first 28c and second transverse surface 28d of a tooth 28 positioned upstream, according to a direction from the coupling end 23 with the handle 26 to the coupling end 21 with the connector 24, are connected to the first 28a and second longitudinal surface 28b of a tooth 28 positioned downstream according to the same direction.

Referring again to Figures 1 to 3, the multi-dose syringe 100 also comprises a flanged dose-setting sleeve 30 and a dose-setting ring 40, which cooperate with each other, and with the helical indentation 27, to set, and subsequently administer, the desired dose of product.

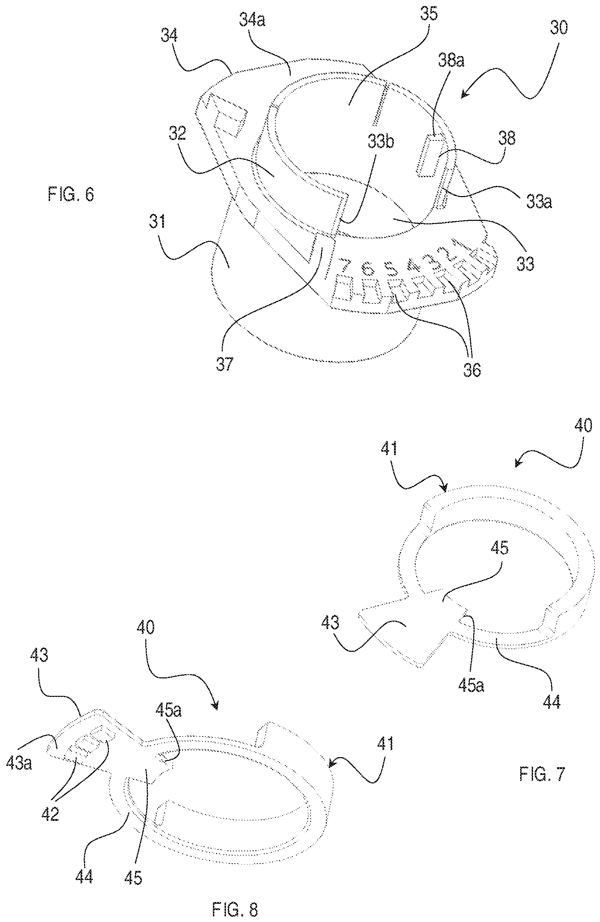

In particular, and as shown in detail in Figure 6, the flanged sleeve 30 comprises a first annular body 31 and a second annular body 32 and a flange 34 interposed between the first annular body 31 and the second annular body 32. The second annular body 32 is provided with an opening 33 and is preferably smaller in diameter than the first annular body 31.

The first annular body 31 is adapted to be fitted irremovably onto the end 11 of the hollow cylindrical body 10 of the multi-dose syringe 100 and the dose-setting ring 40 is adapted to be rotatably mounted on the flanged sleeve 30, more particularly, outside the second annular body 32.

On an inner surface 35 of the second annular body 32, in a position suitably designed for obtaining the desired pre-set doses, specifically in proximity to a side wall 33a of the opening 33, there is a stop element 38, the function of which is to interrupt the stroke of the plunger 20 inside the hollow cylindrical body 10, during the step of administering the dosed amount of product.

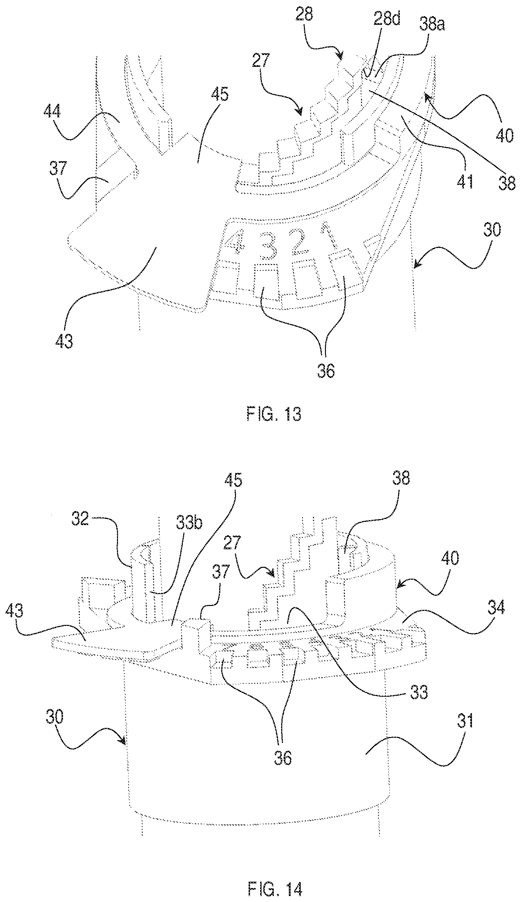

Furthermore, a stop 37 extends from a surface 34a of the flange 34 facing the second annular body 32, precisely in proximity to a side wall 33b of the opening 33, opposite the side wall 33a, whose function is to limit the rotation of the dose-setting ring 40 with respect to the flanged dose-setting sleeve 30, thereby determining the maximum amount of product that can be administered. In addition, and as will be described below with reference to Figure 13, the stop 37 allows the dose-setting ring 40 to be excluded from cooperation with the flanged dose-setting sleeve 30, so that the plunger 20 can rotate freely to administer a desired, and not pre-set, dose of liquid or semiliquid product.

On the surface 34a of the flange 34, there is also a series of interference housing seats 36 for one or more teeth 42 (see Figure 8) formed in the dose-setting ring 40. If the number of teeth 42 exceeds one, they are preferably arranged like an arc of a circle, the centre of which coincides with the longitudinal axis of the multi-dose syringe 100. Thanks to the interference coupling between the seats 36 and the teeth 42, any relative movement between teeth 42 and seats 36 is advantageously prevented, so that the user can set the dose to be dispensed in a way that cannot be altered unintentionally, e.g. as a result of an accidental shock to the multi-dose syringe 100, e.g. by the animal to which the dose is to be administered.

Furthermore, and as will be disclosed in detail in the following of the present description, the purpose of the seats 36 in the flanged sleeve 30 and the tooth/teeth 42 in the ring 40 is to engage with each other, thereby determining, uniquely and repeatably over time, the dose of product to be administered. To this end, progressive numbers, one for each seat, are preferably marked at the seats 36 of the flanged sleeve 30, numbers 1 to 7 in Figure 6, each indicating a quantity, preferably expressed in millilitres, of product to be dosed and subsequently administered. A different, predetermined dose of product to be administered thus correspond to each seat 36 , and, once the dose is set by coupling between the dose-setting ring 40 and the flanged dose-setting sleeve 30, it remains fixed and can thus be administered precisely and repeatably over time.

As shown in detail in Figures 7 and 8, the dose-setting ring 40 comprises a main body 41, from which a tab 43 extends, which is movable within the opening 33 of the flanged sleeve 30. At a surface 43a of the tab 43 facing the surface 34a of the flange 34 of the flanged sleeve 30 during use, teeth 42 are formed, which, as disclosed above, are configured to be accommodated, with interference, within the respective seats 36 formed on the surface 34a of the flange 34, to set a predetermined and repeatable dose of product to be administered.

Preferably, the ring 40 comprises a portion 44, precisely the portion from which the tab 43 extends outwards, which is thinner, preferably flat and therefore less rigid. This advantageously allows the user to easily lift, by elastically deforming it, the tab 43 to release the teeth 42 from their respective seats 36, if, for example, the dose of product to be administered in a repeatable manner over time is to be changed.

An extension 45 of the tab 43 also extends towards the inside of the dose-setting ring 40 to create an abutment surface 45a for the teeth 28 of the helical indentation 27 formed on the outer surface 25 of the plunger 20.

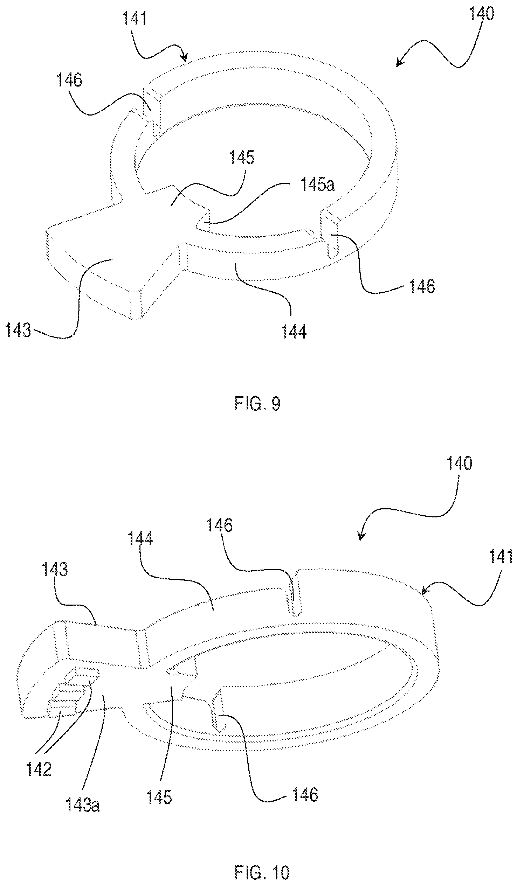

Figures 9 and 10 illustrate an alternative embodiment of a dose-setting ring of the multi-dose syringe 100 according to the present invention. The dose-setting ring, generally indicated by reference number 140, differs from the dose-setting ring 40 described above with reference to Figures 7 and 8, in that it provides, instead of a substantially flat portion, at least one, preferably a pair of recesses 146.

The dose-setting ring 140 thus comprises a main body 141, from which a tab 143 extends, which is movable within the opening 33 of the flanged sleeve 30. At a surface 143a of the tab 143 facing the surface 34a of the flange 34 of the flanged sleeve 30 during use, teeth 142 are formed, which are intended to be accommodated, with interference, within the respective seats 36 formed on the surface 34a of the flange 34, to set a predetermined and repeatable dose of product to be administered.

Preferably, the ring 140 comprises a portion 144, precisely the portion from which the tab 143 extends outwards, which is slightly thinner. In addition, in the transition area between the main body 141 and the portion 144, recesses 146 are formed. The recesses 146 constitute localised weakening zones of the ring 140 and therefore advantageously allow the user to easily lift the tab 143 to release the teeth 142 from their respective seats 36, if, for example, the dose of product to be administered in a repeatable manner over time is to be changed.

An extension 145 of the tab 143 also extends towards the inside of the dose-setting ring 140 thereby creating an abutment surface 145a for the teeth 28 of the helical indentation 27 formed on the outer surface 25 of the plunger 20.

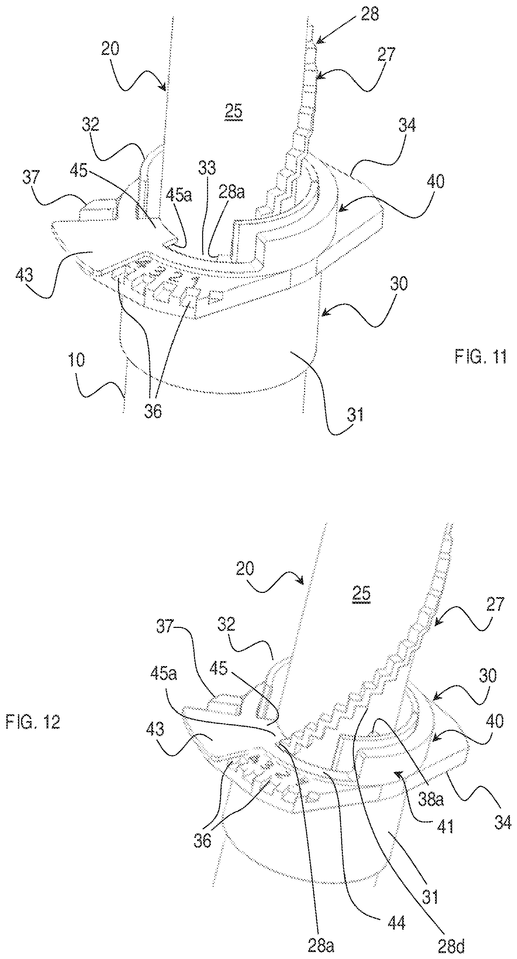

With reference to Figures 11 to 13, a method of multiple dosing a liquid or semiliquid product and subsequently administration of multiple doses, performed using the multi-dose syringe 100 according to the present invention will now be described. The dose-setting ring shown is the ring 40, described above and illustrated in Figures 7 and 8, but it is understood that a similar argument applies when using the dose-setting ring 140 illustrated in Figures 9 and 10.

It is assumed, for example, that the user wishes to administer a precise and repeated dose of the product equal to 5 ml. In this case, the user must, first of all, set the desired dose and, to this end, and as shown in Figure 11, fit the tooth 42 of the ring 40 into the seat 36 marked with the number 5. At this point, the dose has been set and cannot be changed accidentally, so it can be administered repeatedly over time. In fact, and as disclosed above, an interference fit is established between the tooth 42 and the seat 36, so that the tooth 42 cannot be disengaged, unless intentionally. In addition, the elastic force due to the deformation of the dose-setting ring 40 keeps the tooth 42 pressed into the seat 36.

Once the dose to be administered has been set, the user, using the handle 26, rotates the plunger 20 about the longitudinal axis X thereof, until the longitudinal surface 28a of a tooth 28 of the helical indentation 27 abuts against the abutment surface 45a of the portion 45 of the tab 43 of the dose-setting ring 40. This operational condition is shown in Figure 12. The rotation of the plunger 20 about the longitudinal axis X thereof is guaranteed by the presence of the connector 24 interposed between the end 21 of the plunger 20 and the rubber 22. In other words, the connector 24, integral with the plunger 20, rotates inside the rubber 22 stationary inside the hollow cylindrical body 10, when the user rotates the plunger 20.

At this point the set dose of product can be administered precisely and repeatably. To this end the user, if present, removes the plug 15 from the spout 13 of the hollow cylindrical body 10. Then, still using the handle 26, he presses the plunger 20 inside the hollow cylindrical body 10 until the second transverse surface 28d of a tooth 28 of the helical indentation 27 abuts against the stop element 38 and more precisely against the surface 38a of the flanged dose-setting sleeve 30. This causes the outlet of the dose of product previously set by the opening 14 of the spout 13. This operational condition is shown in Figure 13.

It is now assumed that, at a later time, the user desires to dispense the same product dose previously set, to the same animal or to a different animal with the same weight or needing the same product dose.

In this case, the user merely has to repeat the above-described operations of rotating the plunger 20 about the longitudinal axis X thereof until the longitudinal surface 28a of a tooth 28 abuts against the abutment surface 45a of the tab 43, and of pressing the plunger 20 inside the hollow cylindrical body 10 until the transverse surface 28d of a tooth 28 abuts against the stop element 38 and against the upper surface of the flanged sleeve 30.

As a result, if the dose to be administered does not vary, it is not necessary to move the dose-setting ring 40 to engage its teeth 42 in another seat 36 of the flanged sleeve 30, but it is sufficient to rotate the plunger 20. The amplitude of rotation of the plunger 20 then determines the amount of the dose to be administered. In other words, if the dose does not vary, one gesture, i.e. rotating the plunger 20, is sufficient to set the dose to be administered. There is therefore no possibility of error, and the gesture to be performed is very simple. In addition, there are no external factors that can cause the set dose to vary, as the plunger 20 and the flanged sleeve 30 are held by the user and the ring 40 is locked in place on the flanged sleeve 30.

Otherwise, it is now assumed that the user wishes to administer a precise, repeated over time dose of product different from that previously set on the dosing syringe 100, e.g. a dose of 2 ml.

In this case, he must release the ring 40 from the flanged sleeve 30 by grasping the tab 43 and lifting it up. This operation is facilitated by the fact that tab 43 is formed in the thinner, and therefore more flexible, portion 44 of the ring 40. The thickness of the portion 44 is preferably comparable to the thickness of tab 43.

Once the ring 40 has been released from the flanged sleeve 30, the user rotates the ring 40 with respect to the flanged sleeve 30 until at least one tooth 42 is at the seat 36 marked with the number 2, and then lowers the ring 40, so as to engage the tooth 42 in the seat 36.

Once the repeated dose to be dispensed has been set, the user, using the handle 26, rotates the plunger 20 about the longitudinal axis X thereof, until the longitudinal surface 28a of a tooth 28 of the helical indentation 27 abuts against the abutment surface 45a of the portion 45 of the tab 43 of the dose-setting ring 40.

Then, still using the handle 26, the user presses the plunger 20 inside the hollow cylindrical body 10 until the transverse surface 28d of a tooth 28 abuts against an upper surface 38a of the stop element 38 of the flanged dose-setting sleeve 30. This causes the outlet of the dose of product previously set from the opening 14 of the spout 13.

Should the user wish to administer, on a one-off basis, a dose other than the pre-set one, he need not release the tooth 42 from the seat 36 in order to couple it to the seat corresponding to the new dose. Otherwise, the user can simply position a tooth 28 of the helical indentation in such a way that its two longitudinal surfaces 28a and 28b are in alignment, or substantial alignment, with the seat corresponding to the dose to be administered on a one-off basis. If the two longitudinal surfaces 28a and 28b do not align with a seat 36 of the flange 34, but are positioned between two seats 36, e.g. the seats 36 marked by the numbers 3 and 4, corresponding to a 3 ml dose and a 4 ml dose respectively, then the dose that is administered will be that of the seat 36 closest to the tooth 28. To administer the manually set dose, it is sufficient to press the plunger 20.

Finally, it is assumed that a user wishes to use the multi-dose syringe 100 to administer many different doses, without the need to set a predetermined dose that can be repeated over time. In such a case, and as shown in detail in Figure 14, it is sufficient to move the tab 43 of the ring 40, beyond the stop 37, so that the extension 45 is blocked between the stop and the side wall 33b of the opening 33 of the flanged dose-setting sleeve 30, and rotate the plunger 20 according to the animal to be treated, setting the desired dose using the numbers on the flange 34. In this case, the stop 37 and the side wall 33b of the opening 33 are spaced apart by an amount equal to accommodate the extension 45 of the tab 43 of the ring 40.

The aforementioned mode of operation, although less precise than the one involving the use of the ring for repeated doses, is more flexible in terms of dose choice, while still retaining the advantage of immediacy in dose preparation. In fact, a simple rotation of the plunger 20 is sufficient until the longitudinal surfaces 28a and 28b of any tooth 28 are aligned with a number marked on the flange 34 of the flanged dose-setting sleeve 30 and corresponding to a given dose. Once again, if the two longitudinal surfaces 28a and 28b do not align with a seat 36 of the flange 34, but are positioned between two seats 36, e.g. the seats 36 marked by the numbers 3 and 4, corresponding to a 3 ml dose and a 4 ml dose respectively, then the dose that is administered will be that of the seat 36 closest to the tooth 28. This is to ensure that there are no overdoses.

From the above description the features of the syringe and administration method of product doses according to the present invention, as well as the advantages thereof, are evident.

In particular, the multi-dose syringe and the method of multiple dosing a liquid or semiliquid product make it possible to prevent administration errors, since the user does not have to set the dose to be administered at each administration, thus being able, advantageously, to delegate the administration operation to a third party, without the need for special instructions. In addition, the multi-dose syringe prevents the animal causing dosing errors due to moving around. It is also noted that the multi-dose syringe according to the invention can also be used to administer one-off doses or different doses that are not repeated over time.

The invention thus conceived is susceptible to several modifications and variations, all falling within the scope of the invention as defined in the attached claims.

## Claims

1. Multi-dose syringe (100) comprising:
- a hollow cylindrical body (10) configured to contain a product to be administered in a dosed manner and from which a spout (13) extends which is provided with a product outlet opening (14), and a plunger (20), sliding inside the hollow cylindrical body (10);
**wherein** a helical indentation (27) is formed on an outer surface (25) of the plunger (20), which comprises a plurality of teeth (28), each having a first (28a) and a second longitudinal surface (28b) and a first (28c) and a second (28d) transverse surface extending, parallel to each other, orthogonally from a respective longitudinal surface (28a, 28b) and in that the syringe further comprises a flanged dose-setting sleeve (30), irremovably mounted on one end (11) of the hollow cylindrical body (10) for the entry of the plunger (20), and a dose-setting ring (40; 140) mounted on the flanged dose-setting sleeve (30) and rotatable with respect thereto and with respect to the plunger (20),
**characterised in that**
on facing surfaces (34a, 43a) of the flanged sleeve (30) and of the ring (40; 140) a series of seats (36) and at least one tooth (42; 142) configured to be housed with interference in a respective seat (36) are obtained, respectively, and wherein the plunger (20) is rotatable with respect to the hollow cylindrical body (10) until the first longitudinal surface (28a) of a tooth (28) abuts against an abutment surface (45a; 145a) of the ring (40; 140), projecting towards the inside of the hollow cylindrical body (10), so as to set a predetermined dose of the product to be administered.

2. Multi-dose syringe (100) according to claim 1, wherein the flanged dose-setting sleeve (30) comprises a first annular body (31) for mounting the flanged sleeve (30) on the end (11) of the hollow cylindrical body (10) for the entry of the plunger (20), a second annular body (32) provided with an opening (33), and a flange (34) interposed between the first annular body (31) and the second annular body (32), the interference housing seats (36) of the at least one tooth (42; 142) of the dose-setting ring (40; 140) being formed on a surface (34a) of the flange (34).

3. Multi-dose syringe (100) according to claim 2, wherein on an inner surface (35) of the second annular body (32) there is a stop element (38), against which the first transverse surface (28d) of a tooth 28 of the helical indentation (27) abuts, thereby interrupting the stroke of the plunger (20) inside the hollow cylindrical body (10), when administering the dosed amount of product.

4. Multi-dose syringe (100) according to any one of the preceding claims, wherein the plunger (20) comprises a rubber (22) associated with an end (21) of the plunger (20) intended to slide inside the hollow cylindrical body (10), a connector (24) being interposed between the rubber (22) and the end (21) of the plunger (20), to allow a rotation of the plunger (20) with respect to the rubber (22) inside the hollow cylindrical body (10).

5. Multi-dose syringe (100) according to any one of the preceding claims, wherein the dose-setting ring (40; 140) comprises a main body (41; 141), from which a tab (43; 143) extends outwards, the interference housing seats (36) of the at least one tooth (42; 142) of the dose-setting ring (40; 140) being formed at a surface (43a; 143a) of the tab (43; 143).

6. Multi-dose syringe (100) according to claim 5, wherein the tab (43) extends outwards from a portion (44) of the dose-setting ring (40), which is thinner.

7. Multi-dose syringe (100) according to claim 5, wherein the dose-setting ring (140) comprises at least one, preferably two recesses (146) of localised weakening of the ring (144).

8. Multi-dose syringe (100) according to any one of claims 5 to 7, wherein the abutment surface (45a) a tooth (28) of the helical indentation (27) is formed on an extension (45; 145) of the tab (43; 143) facing towards the inside of the dose-setting ring (40; 140).

9. Multi-dose syringe (100) according to any one of claims 2 to 8, wherein a stop (37) extends from a surface (34a) of the flange (34) facing the second annular body (32), adapted to limit the rotation of the dose-setting ring (40) with respect to the flanged dose-setting sleeve (30) or to exclude the dose-setting ring (40) from cooperation with the flanged dose-setting sleeve (30).

10. Multi-dose syringe (100) according to any one of the preceding claims, wherein progressive numbers are marked at the seats (36) of the flanged sleeve (30), one for each seat (36), each indicating an amount of product to be administered in a dosed manner.

11. Multi-dose syringe (100) according to any one of the preceding claims, wherein at another end (23) of the plunger (20) there is a handle (26) preferably having a faceted outer surface.

12. Multi-dose syringe (100) according to any one of the preceding claims, wherein the spout (13) is preferably formed in one piece with the hollow cylindrical body (10), or is a separate piece subsequently assembled on the hollow cylindrical body (10), and is preferably closed by a plug (15).

13. A non-therapeutic method of multiple dosing a product performed by using a multi-dose syringe (100) according to any one of the preceding claims, the method comprising the following steps of:
- rotating a dose-setting ring (40) with respect to a flanged dose-setting sleeve (30) until at least one tooth (42; 142) of the ring (40) is positioned at a respective seat (36) of the flanged sleeve (30), corresponding to a predetermined volume of product to be dosed;
- interference fitting the tooth (42; 142) and the seat (36); and
- rotating a plunger (20) with respect to the flanged sleeve (30) - ring (40) assembly, until a first longitudinal surface (28a) of a tooth (28) of a helical indentation (27) formed on an outer surface (25) of the plunger (20) abuts against an abutment surface (45a; 145a) of the ring (40; 140), projecting towards the inside of dose-setting ring (40), thereby setting a predetermined dose of the product to be administered.

14. Method according to claim 13, further comprising, downstream of the rotating step of the plunger (20), a step of pressing the plunger (20) towards the inside of the hollow cylindrical body (10), until a second surface (28d) of a tooth (28) of the helical indentation (27) abuts against a stop element (38) formed in the flanged sleeve (30), so as to interrupt the stroke of the plunger (20) inside the hollow cylindrical body (10), when the dosed amount of product is administered.

15. Method according to claim 13 or 14, comprising the following additional steps of setting a new product dose:
- lifting the ring (40; 140) from the flanged sleeve (30), so that the tooth (42; 142) is released from the seat (36);
- rotating the ring (40; 140) with respect to the flanged sleeve (30) until at least one tooth (42; 142) of the ring (40; 140) is positioned at a respective seat (36) of the flanged sleeve (30) corresponding to a new dose to be administered;
- interference fitting the tooth (42; 142) and the seat (36); and
- rotating a plunger (20) with respect to the flanged sleeve (30) - dose-setting ring (40; 140) assembly, until the first longitudinal surface (28a) of a tooth (28) of a helical indentation (27) of the plunger (20) abuts against the abutment surface (45a; 145a) of the ring (40; 140), projecting towards the inside of the dose-setting ring (40; 140), thereby setting a predetermined dose of the product to be administered.

## Patentansprüche

1. Mehrfachdosisspritze (100), aufweisend:
- einen hohlen zylindrischen Körper (10), der dazu eingerichtet ist, ein Produkt zu enthalten, das in einer dosierten Weise zu verabreichen ist, und von dem sich ein Auslauf (13) erstreckt, der mit einer Produktauslassöffnung (14) und einem Kolben (20) versehen ist, der innerhalb des hohlen zylindrischen Körpers (10) gleitet;
wobei eine spiralförmige Vertiefung (27) an einer Außenfläche (25) des Kolbens (20) ausgebildet ist, die eine Vielzahl von Zähnen (28) aufweist, die jeweils eine erste (28a) und eine zweite Längsfläche (28b) und eine erste (28c) und eine zweite (28d) Querfläche aufweisen, die sich parallel zueinander orthogonal von einer jeweiligen Längsfläche (28a, 28b) erstrecken, und dadurch, dass die Spritze ferner eine geflanschte Dosiseinstellhülse (30), die unentfernbar an einem Ende (11) des hohlen zylindrischen Körpers (10) für den Eintritt des Kolbens (20) angebracht ist, und einen Dosiseinstellring (40; 140) aufweist, der an der geflanschten Dosiseinstellhülse (30) angebracht ist und in Bezug darauf und in Bezug auf den Kolben (20) drehbar ist,
**dadurch gekennzeichnet, dass**
an sich gegenüberliegenden Flächen (34a, 43a) der geflanschten Hülse (30) und des Rings (40; 140) jeweils eine Reihe von Sitzen (36) und mindestens ein Zahn (42; 142) erhalten werden, der dazu eingerichtet ist, mit Übermaß in einem jeweiligen Sitz (36) aufgenommen zu werden, und wobei der Kolben (20) in Bezug auf den hohlen zylindrischen Körper (10) drehbar ist, bis die erste Längsfläche (28a) eines Zahns (28) an einer Anlagefläche (45a; 145a) des Rings (40; 140) anliegt, die in Richtung des Inneren des hohlen zylindrischen Körpers (10) vorsteht, um eine vorbestimmte Dosis des zu verabreichenden Produkts einzustellen.

2. Mehrfachdosisspritze (100) nach Anspruch 1, wobei die geflanschte Dosiseinstellhülse (30) einen ersten ringförmigen Körper (31) zum Anbringen der geflanschten Hülse (30) an dem Ende (11) des hohlen zylindrischen Körpers (10) für den Eintritt des Kolbens (20), einen zweiten ringförmigen Körper (32), der mit einer Öffnung (33) versehen ist, und einen Flansch (34) aufweist, der zwischen dem ersten ringförmigen Körper (31) und dem zweiten ringförmigen Körper (32) angeordnet ist, wobei die Übermaßaufnahmesitze (36) des mindestens einen Zahns (42; 142) des Dosiseinstellrings (40; 140) an einer Fläche (34a) des Flanschs (34) ausgebildet sind.

3. Mehrfachdosisspritze (100) nach Anspruch 2, wobei an einer Innenfläche (35) des zweiten ringförmigen Körpers (32) ein Anschlagelement (38) vorhanden ist, an dem die erste Querfläche (28d) eines Zahns 28 der spiralförmigen Vertiefung (27) anliegt, wodurch der Hub des Kolbens (20) innerhalb des hohlen zylindrischen Körpers (10) unterbrochen wird, wenn die dosierte Menge des Produkts verabreicht wird.

4. Mehrfachdosisspritze (100) nach einem der vorhergehenden Ansprüche, wobei der Kolben (20) einen Gummi (22) aufweist, der einem Ende (21) des Kolbens (20) zugeordnet ist, das dazu bestimmt ist, innerhalb des hohlen zylindrischen Körpers (10) zu gleiten, wobei ein Verbinder (24) zwischen dem Gummi (22) und dem Ende (21) des Kolbens (20) angeordnet ist, um eine Drehung des Kolbens (20) in Bezug auf den Gummi (22) innerhalb des hohlen zylindrischen Körpers (10) zu ermöglichen.

5. Mehrfachdosisspritze (100) nach einem der vorhergehenden Ansprüche, wobei der Dosiseinstellring (40; 140) einen Hauptkörper (41; 141) aufweist, von dem sich eine Lasche (43; 143) nach außen erstreckt, wobei die Übermaßaufnahmesitze (36) des mindestens einen Zahns (42; 142) des Dosiseinstellrings (40; 140) an einer Fläche (43a; 143a) der Lasche (43; 143) ausgebildet sind.

6. Mehrfachdosisspritze (100) nach Anspruch 5, wobei sich die Lasche (43) von einem Abschnitt (44) des Dosiseinstellrings (40), der dünner ist, nach außen erstreckt.

7. Mehrfachdosisspritze (100) nach Anspruch 5, wobei der Dosiseinstellring (140) mindestens eine, vorzugsweise zwei Aussparungen (146) mit lokaler Schwächung des Rings (144) aufweist.

8. Mehrfachdosisspritze (100) nach einem der Ansprüche 5 bis 7, wobei die Anlagefläche (45a) eines Zahns (28) der spiralförmigen Vertiefung (27) an einer Verlängerung (45; 145) der Lasche (43; 143) ausgebildet ist, die der Innenseite des Dosiseinstellrings (40; 140) zugewandt ist.

9. Mehrfachdosisspritze (100) nach einem der Ansprüche 2 bis 8, wobei sich ein Anschlag (37) von einer Fläche (34a) des Flanschs (34) erstreckt, die dem zweiten ringförmigen Körper (32) zugewandt ist, der dazu ausgelegt ist, die Drehung des Dosiseinstellrings (40) in Bezug auf die mit Flansch versehene Dosiseinstellhülse (30) zu begrenzen oder den Dosiseinstellring (40) vom Zusammenwirken mit der mit Flansch versehenen Dosiseinstellhülse (30) auszuschließen.

10. Mehrfachdosisspritze (100) nach einem der vorhergehenden Ansprüche, wobei fortlaufende Zahlen an den Sitzen (36) der mit Flansch versehenen Hülse (30) markiert sind, eine für jeden Sitz (36), wobei jede eine Menge eines Produkts angibt, das auf eine dosierte Weise verabreicht werden soll.

11. Mehrfachdosisspritze (100) nach einem der vorhergehenden Ansprüche, wobei sich an einem anderen Ende (23) des Kolbens (20) ein Griff (26) befindet, der vorzugsweise eine facettierte Außenfläche aufweist.

12. Mehrfachdosisspritze (100) nach einem der vorhergehenden Ansprüche, wobei der Auslauf (13) vorzugsweise in einem Stück mit dem hohlen zylindrischen Körper (10) ausgebildet ist oder ein separates Stück ist, das anschließend an dem hohlen zylindrischen Körper (10) montiert wird, und vorzugsweise durch einen Stopfen (15) verschlossen wird.

13. Verfahren zur Mehrfachdosierung eines Produkts, das unter Verwendung einer Mehrfachdosisspritze (100) nach einem der vorhergehenden Ansprüche durchgeführt wird, wobei das Verfahren die folgenden Schritte aufweist:
- Drehen eines Dosiseinstellrings (40) in Bezug auf eine mit Flansch versehene Dosiseinstellhülse (30), bis mindestens ein Zahn (42; 142) des Rings (40) an einem jeweiligen Sitz (36) der mit Flansch versehenen Hülse (30) positioniert ist, der einem vorbestimmten Volumen eines zu dosierenden Produkts entspricht;
- Übermaßpassen des Zahns (42; 142) und des Sitzes (36); und
- Drehen eines Kolbens (20) in Bezug auf die Anordnung aus mit Flansch versehener Hülse (30) und Ring (40), bis eine erste Längsfläche (28a) eines Zahns (28) einer spiralförmigen Vertiefung (27), die an einer Außenfläche (25) des Kolbens (20) ausgebildet ist, an einer Anlagefläche (45a; 145a) des Rings (40; 140) anliegt, die in Richtung des Inneren des Dosiseinstellrings (40) vorsteht, wodurch eine vorbestimmte Dosis des zu verabreichenden Produkts eingestellt wird.

14. Verfahren nach Anspruch 13, ferner aufweisend, nach dem Schritt des Drehens des Kolbens (20), einen Schritt des Drückens des Kolbens (20) in Richtung des Inneren des hohlen zylindrischen Körpers (10), bis eine zweite Fläche (28d) eines Zahns (28) der spiralförmigen Vertiefung (27) an einem Anschlagelement (38) anliegt, das in der mit Flansch versehenen Hülse (30) ausgebildet ist, um den Hub des Kolbens (20) innerhalb des hohlen zylindrischen Körpers (10) zu unterbrechen, wenn die dosierte Menge des Produkts verabreicht wird.

15. Verfahren nach Anspruch 13 oder 14, aufweisend die folgenden zusätzlichen Schritte des Einstellens einer neuen Produktdosis:
- Anheben des Rings (40; 140) von der mit Flansch versehenen Hülse (30), so dass der Zahn (42; 142) von dem Sitz (36) freigegeben wird;
- Drehen des Rings (40; 140) in Bezug auf die mit Flansch versehene Hülse (30), bis mindestens ein Zahn (42; 142) des Rings (40; 140) an einem jeweiligen Sitz (36) der mit Flansch versehenen Hülse (30) positioniert ist, der einer neuen zu verabreichenden Dosis entspricht;
- Übermaßpassen des Zahns (42; 142) und des Sitzes (36); und
- Drehen eines Kolbens (20) in Bezug auf die Anordnung aus mit Flansch versehener Hülse (30) und Dosiseinstellring (40; 140), bis die erste Längsfläche (28a) eines Zahns (28) einer spiralförmigen Vertiefung (27) des Kolbens (20) an der Anlagefläche (45a; 145a) des Rings (40; 140) anliegt, die in Richtung des Inneren des Dosiseinstellrings (40; 140) vorsteht, wodurch eine vorbestimmte Dosis des zu verabreichenden Produkts eingestellt wird.

## Revendications

1. - Seringue multidose (100) comprenant :
- un corps cylindrique creux (10) configuré pour contenir un produit à administrer d'une manière dosée et à partir duquel s'étend un bec verseur (13) qui comporte une ouverture de sortie de produit (14), et un piston (20), coulissant à l'intérieur du corps cylindrique creux (10) ;
dans laquelle une indentation hélicoïdale (27) est formée sur une surface externe (25) du piston (20), qui comprend une pluralité de dents (28), chacune ayant une première (28a) et une seconde surface longitudinale (28b) et une première (28c) et une seconde surface transversale (28d) s'étendant, parallèlement l'une à l'autre, orthogonalement à partir d'une surface longitudinale respective (28a, 28b), et dans laquelle la seringue comprend en outre un manchon de réglage de dose à bride (30), monté de manière inamovible sur une extrémité (11) du corps cylindrique creux (10) pour l'entrée du piston (20), et un anneau de réglage de dose (40 ; 140) monté sur le manchon de réglage de dose à bride (30) et apte à tourner par rapport à celui-ci et par rapport au piston (20),
**caractérisée par le fait que**, sur des surfaces en regard (34a, 43a) du manchon à bride (30) et de l'anneau (40 ; 140), une série de sièges (36) et au moins une dent (42 ; 142) configurée pour être logée avec serrage dans un siège respectif (36) sont obtenus, respectivement, et dans laquelle le piston (20) est rotatif par rapport au corps cylindrique creux (10) jusqu'à ce que la première surface longitudinale (28a) d'une dent (28) vienne en butée contre une surface de butée (45a ; 145a) de l'anneau (40 ; 140), faisant saillie vers l'intérieur du corps cylindrique creux (10), de manière à régler une dose prédéterminée du produit à administrer.

2. - Seringue multidose (100) selon la revendication 1, dans laquelle le manchon de réglage de dose à bride (30) comprend un premier corps annulaire (31) pour le montage du manchon à bride (30) sur l'extrémité (11) du corps cylindrique creux (10) pour l'entrée du piston (20), un second corps annulaire (32) comportant une ouverture (33), et une bride (34) interposée entre le premier corps annulaire (31) et le second corps annulaire (32), les sièges de logement avec serrage (36) de l'au moins une dent (42 ; 142) de l'anneau de réglage de dose (40 ; 140) étant formés sur une surface (34a) de la bride (34).

3. - Seringue multidose (100) selon la revendication 2, dans laquelle, sur une surface interne (35) du second corps annulaire (32), il y a un élément d'arrêt (38), contre lequel vient buter la première surface transversale (28d) d'une dent (28) de l'indentation hélicoïdale (27), permettant ainsi d'interrompre la course du piston (20) à l'intérieur du corps cylindrique creux (10), lors de l'administration de la quantité dosée de produit.

4. - Seringue multidose (100) selon l'une quelconque des revendications précédentes, dans laquelle le piston (20) comprend un caoutchouc (22) associé à une extrémité (21) du piston (20) destinée à coulisser à l'intérieur du corps cylindrique creux (10), un connecteur (24) étant interposé entre le caoutchouc (22) et l'extrémité (21) du piston (20), pour permettre une rotation du piston (20) par rapport au caoutchouc (22) à l'intérieur du corps cylindrique creux (10).

5. - Seringue multidose (100) selon l'une quelconque des revendications précédentes, dans laquelle l'anneau de réglage de dose (40 ; 140) comprend un corps principal (41 ; 141), à partir duquel s'étend vers l'extérieur une languette (43 ; 143), les sièges de logement avec serrage (36) de l'au moins une dent (42 ; 142) de l'anneau de réglage de dose (40 ; 140) étant formés à une surface (43a ; 143a) de la languette (43 ; 143).

6. - Seringue multidose (100) selon la revendication 5, dans laquelle la languette (43) s'étend vers l'extérieur à partir d'une partie (44) de l'anneau de réglage de dose (40), qui est plus mince.

7. - Seringue multidose (100) selon la revendication 5, dans laquelle l'anneau de réglage de dose (140) comprend au moins un, de préférence deux évidements (146) d'affaiblissement localisé de l'anneau (144).

8. - Seringue multidose (100) selon l'une quelconque des revendications 5 à 7, dans laquelle la surface de butée (45a) d'une dent (28) de l'indentation hélicoïdale (27) est formée sur un prolongement (45 ; 145) de la languette (43 ; 143) orienté vers l'intérieur de l'anneau de réglage de dose (40 ; 140).

9. - Seringue multidose (100) selon l'une quelconque des revendications 2 à 8, dans laquelle une butée (37) s'étend à partir d'une surface (34a) de la bride (34) tournée vers le second corps annulaire (32), apte à limiter la rotation de l'anneau de réglage de dose (40) par rapport au manchon de réglage de dose à bride (30) ou à exclure l'anneau de réglage de dose (40) d'une coopération avec le manchon de réglage de dose à bride (30).

10. - Seringue multidose (100) selon l'une quelconque des revendications précédentes, dans laquelle des numéros progressifs sont marqués sur les sièges (36) du manchon à bride (30), un pour chaque siège (36), chacun indiquant une quantité de produit à administrer d'une manière dosée.

11. - Seringue multidose (100) selon l'une quelconque des revendications précédentes, dans laquelle, à une autre extrémité (23) du piston (20), il y a une poignée (26) ayant de préférence une surface externe à facettes.

12. - Seringue multidose (100) selon l'une quelconque des revendications précédentes, dans laquelle le bec verseur (13) est, de préférence, formé d'une seule pièce avec le corps cylindrique creux (10), ou est une pièce distincte assemblée ultérieurement sur le corps cylindrique creux (10), et est de préférence fermé par un bouchon (15).

13. - Procédé non thérapeutique de dosage multiple d'un produit réalisé à l'aide d'une seringue multidose (100) selon l'une quelconque des revendications précédentes, le procédé comprenant les étapes suivantes consistant à :
- faire tourner un anneau de réglage de dose (40) par rapport à un manchon de réglage de dose à bride (30) jusqu'à ce qu'au moins une dent (42 ; 142) de l'anneau (40) soit positionnée à un siège respectif (36) du manchon à bride (30), correspondant à un volume prédéterminé de produit à doser ;
- ajuster de manière serrée la dent (42 ; 142) et le siège (36) ; et
- faire tourner un piston (20) par rapport à l'ensemble manchon à bride (30) - anneau (40), jusqu'à ce qu'une première surface longitudinale (28a) d'une dent (28) d'une indentation hélicoïdale (27) formée sur une surface externe (25) du piston (20) vienne en butée contre une surface de butée (45a ; 145a) de l'anneau (40 ; 140), faisant saillie vers l'intérieur de la bague de réglage de dose (40), réglant ainsi une dose prédéterminée du produit à administrer.

14. - Procédé selon la revendication 13, comprenant en outre, en aval de l'étape de rotation du piston (20), une étape de pression du piston (20) vers l'intérieur du corps cylindrique creux (10), jusqu'à ce qu'une seconde surface (28d) d'une dent (28) de l'indentation hélicoïdale (27) vienne en butée contre un élément d'arrêt (38) formé dans le manchon à bride (30), de manière à interrompre la course du piston (20) à l'intérieur du corps cylindrique creux (10), lorsque la quantité dosée de produit est administrée.

15. - Procédé selon la revendication 13 ou 14, comprenant les étapes supplémentaires suivantes de réglage d'une nouvelle dose de produit :
- soulever l'anneau (40 ; 140) à partir du manchon à bride (30), de telle sorte que la dent (42 ; 142) soit libérée du siège (36) ;
- faire tourner l'anneau (40 ; 140) par rapport au manchon à bride (30) jusqu'à ce qu'au moins une dent (42 ; 142) de l'anneau (40 ; 140) soit positionnée à un siège (36) respectif du manchon à bride (30) correspondant à une nouvelle dose à administrer ;
- ajuster de manière serrée la dent (42 ; 142) et le siège (36) ; et
- faire tourner un piston (20) par rapport à l'ensemble manchon à bride (30) - anneau de réglage de dose (40 ; 140), jusqu'à ce que la première surface longitudinale (28a) d'une dent (28) d'une indentation hélicoïdale (27) du piston (20) vienne en butée contre la surface de butée (45a ; 145a) de l'anneau (40 ; 140), faisant saillie vers l'intérieur de l'anneau de réglage de dose (40 ; 140), réglant ainsi une dose prédéterminée du produit à administrer.
